# EUROPÄISCHE PATENTSCHRIFT

(11) **EP 3 843 698 B1**
(45) Veröffentlichungstag und Bekanntmachung des Hinweises auf die Patenterteilung: **27.09.2023**
(21) Anmeldenummer: 19728396.3
(22) Anmeldetag: 31.05.2019
(51) Int. Cl.: A61K 8/44, A61K 8/46, A61K 8/60, A61Q 19/10

(54) **MILDE REINIGUNGSZUBEREITUNG**
MILD CLEANING PREPARATION
PRÉPARATION NETTOYANTE DOUCE

(30) Priorität: 29.08.2018 DE 102018214571
(43) Veröffentlichungstag der Anmeldung: 07.07.2021
(73) Patentinhaber: Beiersdorf AG, 20253 Hamburg (DE)
(72) Erfinder: NILSSON, Jan, 22177 Hamburg (DE)
(74) Vertreter: Beiersdorf AG
(86) Internationale Anmeldenummer: PCT/EP2019/064167
(87) Internationale Veröffentlichungsnummer: WO 2020/043337

(56) Entgegenhaltungen:
- CN-A- 103 381 137
- RU-C1- 2 657 805
- DATABASE GNPD [Online] MINTEL; 6. Juli 2018 (2018-07-06), anonymous: "Body Cleanser", XP055606943, gefunden im www.gnpd.com Database accession no. 5799217
- DATABASE GNPD [Online] MINTEL; 8. Juni 2016 (2016-06-08), anonymous: "Pure Feminine Cleanser", XP055606948, gefunden im www.gnpd.com Database accession no. 4047565
- DATABASE GNPD [Online] MINTEL; 20. April 2016 (2016-04-20), anonymous: "Balancing Cleansing Cream", XP55606958, gefunden im www.gnpd.com Database accession no. 3897143
- DATABASE GNPD [Online] MINTEL; 8. Februar 2012 (2012-02-08), anonymous: "Creamy Body Wash", XP055607193, gefunden im www.gnpd.com Database accession no. 1722577
- DATABASE GNPD [Online] MINTEL; 17. April 2018 (2018-04-17), anonymous: "Moisturizing Foam Cleanser", XP55606947, gefunden im www.gnpd.com Database accession no. 5591335
- Anonymous: "COMPRITOL 88 CG ATO", , 1. Januar 2017 (2017-01-01), XP55607197, Gefunden im Internet: URL:https://www.i9magistralshop.com.br/com pritol-88-cg-ato-03886 [gefunden am 2019-07-19]
- Dr Tony Gough: "Formulating High Performance, Sulfate- Free Cleansing Products Content", , 16. April 2015 (2015-04-16), XP55607134, Gefunden im Internet: URL:https://www.in-cosmetics.com/RXUK/RXUK _InCosmetics/2015-Website/Documents/in-cos 15,IS,T3,D3,Formulating%20high%20performan ce,%20sulfate-free%20cleansing%20products, Dr.%20Tony%20Gough.pdf?v=63565398552404303 7 [gefunden am 2019-07-19]
- Geert De Lathauwer ET AL: "THICKENING OF FOAMING COSMETIC FORMULATIONS", , 23. Juni 2004 (2004-06-23), XP055250896, Gefunden im Internet: URL:http://www.firp.ula.ve/archivos/materi al_web_4xx/04_CESIO_DeLathauwer_154.pdf [gefunden am 2016-02-17]

## Beschreibung

Kosmetische Produkte dienen im Allgemeinen nicht nur dazu schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut eingesetzt.

Insbesondere Reinigungszubereitungen für Babys müssen erhöhte Anforderungen für eine sanfte und angenehme Reinigung erfüllen, so dass es bei Ihrer Anwendung zu keinen Hautirritationen und/oder einem brennen bei Augenkontakt kommt. Um diesen Anforderungen genüge zu tragen besteht ein ständiger Bedarf an Formulierungen, welche besonders zur Reinigung von Babyhaut geeignet sind.

Wie unter anderem die EP 1010422 A2 ausführt, ist es für Reinigungszubereitungen für Babys ebenfalls entscheidend, dass die Reinigungszubereitung ein Verdickersystem enthält, um die Möglichkeit zu minimieren, dass das Produkt in die Augen fließt. Betrachtet man jedoch die derzeit eingesetzten Verdickersysteme für Reinigungszubereitungen, so weisen diese eine Vielzahl von Nachteilen auf.

Generell ist es entscheidend, dass die Reinigungszubereitungen eine Viskosität im Bereich von 1000 bis 4000 mPa·s, bevorzugt 1500 bis 2500 mPa·s aufweisen. So wird sichergestellt, dass diese sich auf der Haut verteilen lassen, ohne dass diese abrutschen. Gleichzeitig ist eine Entnahme aus Tuben oder Quetschflaschen möglich.

Bekannte Verdickersysteme für Reinigungszubereitungen weisen jedoch insbesondere für den Einsatz auf sensibler Babyhaut erhebliche Nachteile auf, welche im Folgenden dargestellt werden.

Beispielsweise können Reinigungszubereitungen mit PEG-haltigen Tensidsystemen oftmals durch die Zugabe von Salzen oder Elektrolyten verdicket werden. Ein einsetzbares Tensid ist Natriumlaurylethersulfat, vorteilhaft in Kombination mit Betainen. Als Salz kann unter anderem Natriumchlorid eingesetzt werden. Jedoch sind solche Verdickersysteme für den Einsatz in hautfreundlichen Reinigungszubereitungen für Babys ungeeignet, da bekanntermaßen Natriumlaurylethersulfat ein erhöhtes Hautirritationspotential aufweist. Ferner stehen PEG-haltige Substanzen zunehmend in der Kritik, da diese im Verdacht stehen die Haut durchlässiger zu machen, indem sie die natürliche Schutzbarriere der Haut abschwächen. Insbesondere bei Babyprodukte sollte somit auf derartige Substanzen verzichtet werden.

Eine Salzverdickung/Elektrolytverdickung, wie vorstehend für die PEG-haltigen Tenside beschrieben, ist bei ausschließlicher Verwendung von hautverträglichen amphoteren oder nichtionischen Tensiden nicht oder nur sehr begrenzt möglich. Vielmehr führt die Zugabe von Salzen, wie Natriumchlorid, oftmals zu einem schnellen Viskositätsverlust.

Alternativ zu der Salz/Elektrolytverdickung ist es ein standartmäßiges Verfahren, milden Reinigungszubereitungen verdickende Acrylat-basierte Polymere oder weitere verdickende Substanzen zuzusetzen. Typische Verdicker sind beispielsweise PEG-haltige Verdicker, wie PEG-120 Methyl Glucose Dioleat oder PEG-200 Hydrogenated Glyceryl Palmate, welche ebenfalls, wie vorstehend für die PEG-haltigen Tenside beschrieben, in der Kritik stehen und somit nicht eingesetzt werden sollten. Zu den Acrylat-basierten Polymeren zählen Polymere, welche aus Homo- oder Copolymerisation mit Acryl- und/oder Methacrylsäure erhalten werden. Beispiele hierfür sind u.a. Carbomer oder Acrylates Copolymer. Jedoch ist auch der Einsatz dieser Acrylat-basierten Polymere zunehmend in der Kritik, da deren biologische Abbaubarkeit nicht vollständig geklärt ist.

Weiterhin ist es dem Fachmann bekannt die Polymere Xanthan Gum, Cellulosen und Cellulosederivate und/oder Stärken und Stärkederivate als Verdicker einzusetzen. Diese Biopolymere stehen zwar nicht in der Kritik, dass sie biologisch nicht abbaubar sind, weisen bei Einsatz zur Verdickung von Reinigungszubereitungen jedoch erhebliche Nachteile auf. So zeigen derartige verdickende Biopolymere oftmals einen negativen Einfluss auf die mikrobiologische Stabilität, welche nur über einen zusätzlichen Einsatz von Konservierungsmitteln kompensiert werden kann. Ein weiterer negativer Aspekt dieser Biopolymere ist, dass diese oftmals schwankende Mengen an Elektrolyten enthalten. Das hat zur Folge, dass die einzelnen Reinigungszubereitungen nicht gleichmäßig zu produzieren sind und diese abhängig von der Elektrolytmenge in den Rohstoffen nach Produktion oftmals unterschiedliche rheologische Eigenschaften/Viskositäten aufweisen. Somit weisen die produzierten Reinigungszubereitungen aus unterschiedlichen Chargen oftmals unterschiedliche Eigenschaften auf, was bei Massenware zu verhindern ist, da der Konsument gleichbleibende Qualität erwartet.

Ein weiterer negativer Aspekt ist, dass die Polymere Xanthan Gum, Cellulosen und Cellulosederivate und/oder Stärken und Stärkederivate sowie Acrylat-basierte Polymere das Schaumverhalten von Reinigungszubereitungen negativ beeinflussen. So wird bei deren Einsatz das erhaltene Schaumvolumen der Reinigungszubereitung reduziert. Eine Kompensation erfolgt oft mit hohen Konzentrationen an PEG-haltigen Tensiden, wie Natriumlaurylethersulfat, was wiederum zu einem erhöhten Hautirritationspotential führt.

Dementsprechend besteht weiterhin ein Bedarf an Mitteln zur Bereitstellung verdickter opaker/milchiger Reinigungszubereitungen, insbesondere für Babys.

Überraschend für den Fachmann wurde nun gefunden, dass der kombinierte Einsatz von spezifischen Tenside zu einer synergistischen Verdickung führt. Weiterhin zeigte diese spezifische Kombination überraschend nicht die aufgeführten Nachteile der vorstehend beschriebenen Verdickersysteme.

Gegenstand der vorliegenden Erfindung ist daher eine kosmetische Reinigungszubereitung laut Anspruch 1.

Wie die vorliegenden Vergleichsversuche zeigen, zeigt die erfindungsgemäße Kombination eine synergistische Verdickung der Reinigungszubereitung, während bei Einsatz der Einzelkomponenten bzw. der Zweierkombinationen keinen Verdickung zeigen.

Sollten nachfolgend Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der kosmetischen Reinigungszubereitung. Sollten nachfolgend Verhältnisse von Komponenten/Substanzen/Stoffgruppen offenbart werden, so beziehen sich diese Verhältnisse auf Gewichtsverhältnisse der genannten Komponenten/Substanzen/Stoffgruppen.

Werden nachfolgend Gewichtsprozentbereiche für die Bestandteile der kosmetischen Reinigungszubereitung angegeben, so umfasst die Offenbarung der vorliegenden Anmeldung ebenfalls alle Einzelwerte in Schritten von 0,1 Gew.-% innerhalb dieser Gewichtsprozentbereiche.

Werden in dieser Offenbarung Viskositätswerte angegeben, so beziehen sich alle Werte auf eine Messung bei 25°C im 150 ml Rollrandglas mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messsystems wird die Viskosität berechnet. Als Messkörper wird der Messkörper Nr.1 (Artikelnr. 200 0191), geeignet für einen Viskositätsbereich bis 10.000 [mPa·s], Drehzahl Bereich 62,5 min⁻¹, verwendet. Erfolgt keine andere Angabe, so erfolgt die Messung zur Viskosität immer 24h nach Herstellung der Reinigungszubereitung. Das gleiche gilt für alle angegeben Viskositätswerte, welche sich immer auf eine Messung 24h nach Herstellung beziehen, sofern keine andere Zeitangabe angegeben ist.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Erfindungsgemäß vorteilhaft beträgt der Anteil an Lauryl Glucoside in der kosmetischen Reinigungszubereitung von 1 Gew.-% bis 6 Gew.-%, bevorzugt von 1,5 Gew.-% bis 4,5 Gew.-% und insbesondere bevorzugt von 2,5 Gew.-% bis 3,5 Gew.-%, jeweils bezogen auf das Gesamtgewicht der Reinigungszubereitung.

Kommerziell kann Lauryl Glucoside unter dem Handelsnamen Plantacare^{®} 1200 UP von der BASF bezogen werden.

Weiterhin ist es erfindungsgemäß vorteilhaft, wenn der Anteil von Sodium Methyl Cocoyl Taurate von 0,25 Gew.-% bis 4 Gew.-%, bevorzugt von 0,5 Gew.-% bis 2,5 Gew.-% und insbesondere bevorzugt von 1 Gew.-% bis 2 Gew.-%, jeweils bezogen auf das Gesamtgewicht der Reinigungszubereitung, beträgt.

Sodium Methyl Cocoyl Taurate kann unter anderem von der Firma Innospec Performance Chemicals bezogen werden.

Ferner ist es ebenfalls vorteilhaft im Sinne der vorliegenden Erfindung, wenn Cocamidopropylbetaine in einem Anteil von 2 Gew.-% bis 8 Gew.-%, bevorzugt von 3 Gew.- % bis 7 Gew.-% und insbesondere bevorzugt von 4 Gew.-% bis 5 Gew.-%, jeweils bezogen auf das Gesamtgewicht der Reinigungszubereitung, enthalten ist.

Weiterhin ist die erfindungsgemäße kosmetische Reinigungszubereitung vorteilhaft dadurch gekennzeichnet, dass diese einen pH-Wert im Bereich von 4,0 bis 5,5 und bevorzugt von 4,4 bis 4,8 aufweist.

Darüber hinaus ist die erfindungsgemäße kosmetische Reinigungszubereitung bevorzugt dadurch gekennzeichnet, dass diese keine mit Polyethylen Glykol substituierte Substanzen enthält. Somit sind bevorzugt keine PEG-haltigen Substanzen enthalten.

Es ist zwar generell erfindungsgemäß möglich zur weiteren Verdickung der kosmetischen Reinigungszubereitung Acrylat-basierte Polymere zuzusetzen, jedoch ist es bevorzugt auf den Einsatz von Acrylat-basierten Polymeren zu verzichteten, da die gewünschte Verdickung der Zubereitung durch die spezifische Kombination der Tenside erreicht wird. Daher ist die erfindungsgemäße kosmetische Reinigungszubereitung bevorzugt dadurch gekennzeichnet, dass die diese keine Acrylat-basierten Polymere enthält.

Es ist ebenfalls erfindungsgemäß möglich zur weiteren Verdickung weitere Polymere auf Basis von Polysacchariden und/oder Stärken der kosmetischen Reinigungszubereitung zuzusetzen, jedoch ist es bevorzugt auf den Einsatz von weitere Polymere auf Basis von Polysacchariden und/oder Stärken zu verzichteten, da die gewünschte Verdickung der Zubereitung durch die spezifische Kombination der Tenside erreicht wird. Daher ist die erfindungsgemäße kosmetische Reinigungszubereitung bevorzugt dadurch gekennzeichnet, dass die diese keine zusätzlichen Polymere auf Basis von Polysacchariden und/oder Stärken enthält.

Weiterhin ist es vorteilhaft im Sinne der vorliegenden Erfindung, wenn die kosmetische Reinigungszubereitung kein Natriumlaurylethersulfat oder Natriumaurylsulfat enthält.

Eine erste vorteilhafte Ausführungsform der vorliegenden Erfindung ist ferner dadurch gekennzeichnet, dass neben den erfindungsgemäßen Tensiden keine weiteren Tenside in der erfindungsgemäßen Reinigungszubereitung enthalten sind. So wurde bei Einsatz der erfindungsgemäßen Tenside bereits eine überraschend gute Reinigungsleistung erzielt. Überraschend war diese erfindungsgemäße Kombination an Tensiden ebenfalls besonders milde zur Haut, so dass die Reinigungszubereitung besonders zur Reinigung von Babyhaut, also bis zu einem Lebensalter von 3 Jahren, geeignet ist.

Wie für die erste Ausführungsform der Erfindung bereits beschrieben, zeigt die erfindungsgemäße Reinigungszubereitung nicht nur eine effektive Reinigungsleistung, sondern ist auch noch besonders schonend für die Haut. Besteht jedoch ein Bedarf, das Reinigungsvermögen der Reinigungszubereitung weiter zu steigern, oder die Menge an gebildetem Schaum bezogen auf das Volumen des Schaums zu erhöhen, so ist es erfindungsgemäß in einer zweiten vorteilhaften Ausführungsform möglich weitere Tenside in der erfindungsgemäßen Reinigungszubereitung einzusetzen. Eine zweite vorteilhafte Ausführungsform der Erfindung ist somit dadurch gekennzeichnet, dass neben den erfindungsgemäßen Tensiden zusätzlich weitere Tenside enthalten sind.

Innerhalb der zweiten vorteilhaften Ausführungsform ist es bevorzugt, wenn als weiteres Tensid Sodium Lauroyl Methyl Isethionate enthalten ist. Ist Sodium Lauroyl Methyl Isethionate enthalten, so beträgt der Anteil von Sodium Lauroyl Methyl Isethionate vorteilhaft von 0,1 Gew.-% bis 1,2 Gew.-% bezogen auf das Gesamtgewicht der Reinigungszubereitung.

Innerhalb der zweiten vorteilhaften Ausführungsform ist es auch bevorzugt, wenn als weiteres Tensid Decyl Glucoside enthalten ist. Ist Decyl Glucoside enthalten, so beträgt der Anteil von Decyl Glucoside vorteilhaft von 0,1 Gew.-% bis 1,5 Gew.-% bezogen auf das Gesamtgewicht der Reinigungszubereitung.

Trotz dass weitere Tenside, wie vorteilhafter weise Decyl Glucoside und/oder Sodium Lauroyl Methyl Isethionate in der zweiten vorteilhaften Ausführungsform der Erfindung enthalten sein dürfen, hat es sich weiterhin gezeigt, dass es dennoch vorteilhaft ist, wenn die Reinigungszubereitung dieser Ausführungsform kein Sodium Cocoylglutamate und/oder kein Sodium Lauroamphoacetate enthält.

Die nachfolgend beschriebenen Merkmale der erfindungsgemäßen Reinigungszubereitung beziehen sich auf die erfindungsgemäße Tensidkombination an sich sowie jeweils auf die zuvor beschriebenen erfindungsgemäßen vorteilhaften Ausführungsformen.

Ferner ist es weiterhin bevorzugt, wenn der Gesamtanteil aller enthaltenen Tenside von 3,25 Gew.-% bis 18 Gew.-%, weiterhin bevorzugt von 5 Gew.-% bis 14 Gew.-% und insbesondere bevorzugt von 7,5 Gew.-% bis 10,5 Gew.-%, jeweils bezogen auf das Gesamtgewicht der kosmetischen Reinigungszubereitung beträgt.

Die erfindungsgemäßen kosmetischen Reinigungszubereitungen können weiterhin vorteilhaft Natriumbenzoat enthalten. Enthält die kosmetische Reinigungszubereitung Natriumbenzoat, so ist es erfindungsgemäß vorteilhaft, wenn der Anteil an Natirumbenzoat von 0,1 Gew.-% bis 0,5 Gew.-% bezogen auf das Gesamtgewicht der Reinigungszubereitung beträgt.

Weiterhin ist es vorteilhaft, wenn die kosmetische Reinigungszubereitung dadurch gekennzeichnet ist, dass diese Natriumsalicylat enthält und der Anteil von Natriumsalicylat bevorzugt von 0,1 Gew.-% bis 0,5 Gew.-% bezogen auf das Gesamtgewicht der Reinigungszubereitung beträgt.

Weiterhin sind vorteilhafte kosmetische Reinigungszubereitungen der Erfindung dadurch gekennzeichnet, dass diese ein oder mehrere Alkandiole mit 6 oder 8 Kohlenstoffatomen enthalten, wobei der Gesamtanteil diese Alkandiole mit 6 oder 8 Kohlenstoffatomen bevorzugt von 0,01 Gew.-% bis 0,4 Gew.-% bezogen auf das Gesamtgewicht der Reinigungszubereitung beträgt.

Zusätzlich ist es vorteilhaft im Sinne der vorliegenden Erfindung, wenn die kosmetische Reinigungszubereitung Glycerin enthält und der Anteil von Glycerin bevorzugt von 0,01 Gew.-% bis 10 Gew.-% bezogen auf das Gesamtgewicht der Reinigungszubereitung beträgt.

Ferner war es für den Fachmann ebenfalls überraschend, dass im Gegensatz zu anderen verdickten Zubereitung die erfindungsgemäße Reinigungszubereitung überaus stabil ist bei Zugabe von bis zu 2,5 Gew.-% Natriumchlorid. Stabil heißt in diesem Zusammenhang, dass sich keine Änderungen der Viskosität der Zubereitung von mehr als ±600 mPa·s einstellt. Dementsprechend sind ebenfalls kosmetische Reinigungszubereitungen bevorzugt, die dadurch gekennzeichnet sind, dass diese kein Natriumchlorid enthalten oder der Anteil von Natriumchlorid maximal 2,5 Gew.-% bezogen auf das Gesamtgewicht der jeweiligen kosmetischen Reinigungszubereitung beträgt.

Es ist ebenfalls erfindungsgemäß von Vorteil, wenn die kosmetische Reinigungszubereitung Octyldodecanol enthält und der Anteil von Octyldodecanol von 0,01 Gew.-% bis 0,3 Gew.-% bezogen auf das Gesamtgewicht der Reinigungszubereitung beträgt. Überraschend wurde gefunden, dass der Einsatz von Octyldodecanol in den vorstehend beschriebenen Konzentrationen in der erfindungsgemäßen Reinigungszubereitung zu einem Anstieg oder gleichbleibender der Viskosität im Vergleich zu Octyldecanol-freien Zubereitung führt, während der Einsatz höherer Konzentration zu einer Reduzierung der Viskosität führt. Weiterhin ist es ebenfalls bevorzugt, wenn die kosmetischen Reinigungszubereitungen Wasser in einem Anteil von 50 Gew.-% bis 94 Gew.-%, weiterhin bevorzugt von 65 Gew.-% bis 92 Gew.-% und insbesondere bevorzugt von 68 Gew.-% bis 90 Gew.-% bezogen auf das Gesamtgewicht der kosmetischen Reinigungszubereitung enthalten.

Im Stand der Technik ist es ebenfalls bekannt sogenannte Trübungsmittel den Reinigungszubereitungen zuzusetzen, um Reinigungszubereitungen mit einem milchigem/ opaken Erscheinungsbild herzustellen. Problematisch ist jedoch der Umstand, dass die konventionellen Trübungsmittel, wie Acrylates Styrene Copolymer, oftmals als Mikroplastik beschrieben werden und so vom Verbraucher nicht gewünscht sind. Ferner kommt es beim Einsatz von Trübungsmittel in kosmetischen Reinigungszubereitung oftmals zu Phasentrennung, da die Zubereitungen die Partikel nicht ausreichen in Schwebe halten können.

Überraschend wurde nun ebenfalls festgestellt, dass wenn eine Reinigungszubereitung mit einem opaken/milchigem Erscheinungsbild bereitgestellt werden soll, die nicht die Nachteile des Standes der Technik aufweist, sich dieses erreichen lässt, indem der kosmetischen Reinigungszubereitung Glyceryl Behenate, Glyceryl Dibehenate und/oder Tribehenin zugesetzt wird.

Die Ausführungsformen der Erfindung enthalten dementsprechend Glyceryl Behenate, Glyceryl Dibehenate und/oder Tribehenin. Der Gesamtanteil von Glyceryl Behenate, Glyceryl Dibehenate und/oder Tribehenin liegt dabei bevorzugt im Bereich von 1 Gew.-% bis 3 Gew.-% bezogen auf das Gesamtgewicht der kosmetischen Reinigungszubereitung. Es kam überraschend zu keinem Viskositätsverlust und zu keiner Phasentrennung innerhalb von 60 Tagen bei Lagerung unter Normalbedingungen. Weiterhin hat sich überraschend für den Fachmann gezeigt, dass die Transparenz der kosmetischen Reinigungszubereitung erhöht werden kann, indem vor Mischen der Bestandteile Sodium Methyl Cocoyl Taurate separat aufgeschmolzen wird, und das flüssige Sodium Methyl Cocoyl Taurate der restlichen Zubereitung zugegeben wird, wobei die restliche Reinigungszubereitung vorteilhaft eine Temperatur im Bereich von 10°C bis 30°C Temperatur aufweist. Weiterhin ist es ebenfalls vorteilhaft, wenn Lauryl Glucoside separat aufgeschmolzen wird und der restlichen Reinigungszubereitung zugegeben wird.

Vorteilhaft ist somit auch ein Verfahren zur Herstellung der erfindungsgemäßen kosmetischen Zubereitung dadurch gekennzeichnet, dass
a) Sodium Methyl Cocoyl Taurate wird aufgeschmolzen bis alle Feststoffe gelöst sind,
b) Lauryl Glucoside wird aufgeschmolzen bis alle Feststoffe gelöst sind, und
c) Beide Tenside zum erhalten der erfindungsgemäßen kosmetischen Reinigungszubereitung zu den restlichen Bestandteilen der Reinigungszubereitung zugegeben werden, wobei die restlichen Bestandteile der Reinigungszubereitung vorteilhaft eine Temperatur im Bereich von 10°C bis 30°C aufweisen.

Die kosmetischen Reinigungszubereitungen gemäß der Erfindung können ferner kosmetische Hilfsstoffe und Wirkstoffe enthalten, wie sie üblicherweise in solchen Zubereitungen verwendet werden, z. B. weitere Wirkstoffe, Konservierungsmittel, Konservierungshelfer, Bakterizide, Lipide, Farbstoffe und Farbpigmente, Verdickungsmittel, anfeuchtende und/oder feuchthaltende Substanzen oder andere übliche Bestandteile einer kosmetischen oder dermatologischen Formulierung wie weitere Polyole, Schaumstabilisatoren, organische Lösungsmittel oder Silikonderivate, sofern der Zusatz die geforderten Eigenschaften hinsichtlich der Stabilität nicht beeinträchtigen oder ausgeschlossen sind.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

In der nachfolgenden Tabelle werden die Beispielrezepturen Bsp.1 bis Bsp.6 aufgeführt, wobei die Beispielrezepturen Bsp.1, Bsp.5 und Bsp.6 keine erfindungsgemäße Kombination offenbaren. Auch die weiteren Beispielrezepturen 2-4, 8-18 sind nicht gemäß der Erfindung, wobei jede jeweils eins der erfindungsgemäßen Tenside nicht enthält. Wie sich zeigt, reicht die jeweilige Zweierkombination nicht aus, um die gewünschte Viskosität zu erreichen. Überraschend zeigt die erfindungsgemäße Kombination jedoch eine Verdickung, so dass eine gewünschte Reinigungszubereitung ohne Einsatz von weiteren verdickenden Polymeren erhalten werden kann. Die Messung der Viskosität erfolgte gemäß der zuvor offenbarten Messmethode.

| **Inhaltsstoffe** | **Bsp.1** | **Bsp.2** | **Bsp.3** | **Bsp.4** | **Bsp.5** | **Bsp.6** |
|---|---|---|---|---|---|---|
| Octyldodecanol | 0,125 | 0,125 | 0,125 | 0,125 | 0,125 | 0,125 |
| Glycerin | 2 | 2 | 2 | 2 | 2 | 2 |
| Sodium Benzoate | 0,45 | 0,45 | 0,45 | 0,45 | 0,45 | 0,45 |
| Cocamidopropyl Betaine | 4,42 | | 4,42 | 4,42 | 4,4 | 4,40 |
| Sodium Methyl Cocoyl Taurate | 1,25 | 1,25 | | 1,25 | 1,25 | 1,25 |
| Lauryl Glucoside | 3,09 | 3,09 | 3,09 | | 3,1 | 3,1 |
| Decyl Glucoside | | | | | 1,0 | 0 |
| Sodium Lauroyl Methyl Isethionate | | | | | | 1,0 |
| Zugabe von Citric Acid zur Einstellung von pH 4,4 - 4,8 | q.s | q.s | q.s. | q.s. | q.s. | q.s. |
| Aqua | ad 100 | ad 100 | ad 100 | ad 100 | ad 100 | ad 100 |
| | | | | | | |
| Gemessene Viskosität 24h nach Herstellung in mPa·s | 2250 | <250 / nicht messbar | <250 / nicht messbar | <250 / nicht messbar | 2550 | 1750 |

Nach Herstellung der Beispielzubereitung Bsp.1 war diese transparent.

Die Transparenz einer Zubereitung wird erfindungsgemäß wie folgt beurteilt:
1. Die Zubereitung wird in eine Küvette (BrandTech^{™} BRAND^{™} UV-Küvette, Einmalküvetten, BrandTech^{™} 759170, Strahlengang 10 mm) gefüllt.
2. An der vom Betrachter abgewandten Seite der Küvette wird ein Zettel mit den Buchstaben "II" in Arial, Schriftgröße 10, befestigt.
3. Sofern der Betrachter mit dem bloßem Auge beim Durchschauen durch die Küvette mit der Flüssigkeit, die Buchstaben "II" getrennt erkennen kann, wird die Zubereitung als transparent bezeichnet.

Es wurde gefunden, dass die Zubereitung des Bsp.1 vollkommen klar ist und die Buchstaben erkannt werden. Weiterhin wurde eine Zubereitung Bsp.7 hergestellt, welche der Zubereitung Bsp.1 einsprach, wobei diese Zubereitung zusätzlich 2 Gew.-% einer Mischung aus Glyceryl Behenate, Glyceryl Dibehenate und Tribehenin im Ausgleich für die äquivalente Menge Wasser enthielt. Es wurde als Mischung aus Glyceryl Behenate, Glyceryl Dibehenate und Tribehenin das Handelsprodukt Compritol 888 CG ATO von der Firma Gattefosse eingesetzt. Die Mischung des Handelsprodukts setzt sich aus 50,0 Gew.-% Glyceryl Behenate, 12,5 Gew.-% Glyceryl Dibehenate und 37,5 Gew.-% Tribehenin zusammen. Durch den Zusatz der Mischung aus Glyceryl Behenate, Glyceryl Dibehenate und Tribehenin konnte eine opake/milchige Zubereitung erhalten werden, welche nicht transparent ist. Es kam überraschend zu keinem Viskositätsverlust und zu keiner Phasentrennung innerhalb von 60 Tagen nach Herstellung der Zubereitung und Lagerung der Zubereitung unter Normalbedingungen.

Weiterhin sind die Reinigungszubereitungen 19 bis 23 der vorliegenden Erfindung überraschend milde, so dass Sie sich besonders gut zur Anwendung auf der Babyhaut eignen.

| **Inhaltsstoffe** | **Bsp. 8** | **Bsp. 9** | **Bsp. 10** | **Bsp. 11** | **Bsp. 12** | **Bsp. 13** | **Bsp. 14** | **Bsp. 15** | **Bsp. 16** |
|---|---|---|---|---|---|---|---|---|---|
| Cocamidopropyl Betaine | 4,42 | 4,40 | 4,50 | 4,40 | 4,50 | 4,00 | 4,42 | 2,50 | 4,50 |
| Sodium Methyl Cocoyl Taurate | 1,25 | 1,20 | 1,20 | 1,50 | 1,20 | 1,25 | 1,35 | 1,50 | 1,00 |
| Lauryl Glucoside | 3,05 | 3,10 | 3,2 | 3,15 | 3,10 | 2,50 | 3,04 | 3,00 | 3,00 |
| Sodium Benzoate | 0,45 | 0,45 | 0,15 | 0,15 | 0,30 | 0,30 | 0,30 | 0,45 | 0,45 |
| Octyldodecanol | | 0,25 | 0,13 | 0,13 | 0,13 | 0,13 | 0,13 | | |
| Parfum | | | | | | | | 0,50 | 0,50 |
| Glycerin | 2,00 | 2,00 | 2,00 | 2,00 | 2,00 | 2,00 | 2,00 | 2,00 | 2,00 |
| Sodium Chloride | | | 0,30 | | | | | | |
| Citric Acid | ad to pH 4,4-4,8 | ad to pH 4,4-4,8 | ad to pH 4,4-4,8 | ad to pH 4,4-4,8 | ad to pH 4,4-4,8 | ad to pH 4,4-4,8 | ad to pH 4,4-4,8 | ad to pH 4,4-4,8 | ad to pH 4,4-4,8 |
| Aqua | ad 100 | ad 100 | ad 100 | ad 100 | ad 100 | ad 100 | ad 100 | ad 100 | ad 100 |

| **Inhaltsstoffe** | **Bsp.17** | **Bsp.18** | **Bsp.19** | **Bsp.20** | **Bsp.21** | **Bsp.22** | **Bsp.23** | | |
|---|---|---|---|---|---|---|---|---|---|
| Cocamidopropyl Betaine | 4,5 | 4,35 | 4,55 | 4,4 | 4,3 | 4,1 | 4,6 | | |
| Glycerin | 2 | 2 | 2 | 2 | 2 | 10 | 2 | | |
| Sodium Methyl Cocoyl Taurate | 1,2 | 1,3 | 1,5 | 1,05 | 1,4 | 1,2 | 1,4 | | |
| Sodium Benzoate | 0,45 | 0,45 | 0,45 | 0,45 | 0,45 | 0,45 | 0,45 | | |
| Parfum | 0,5 | 0,5 | 0,5 | 0,5 | 0,5 | 0,5 | 0,5 | | |
| Lauryl Glucoside | 3,15 | 3,1 | 3,2 | 3,4 | 3,5 | 3,7 | 3,6 | | |
| Hydroxypropyl Guar Hydroxypropyltrimonium Chloride | | | 0,2 | | | | | | |
| Sodium Hydroxide | | | 0,02 | | | | | | |
| Glyceryl Dibehenate + Tribehenin + Glyceryl Behenate¹ | | | 2 | 2 | 2 | 2 | 2 | | |
| Polyquaternium-10 | | | | 0,2 | | | | | |
| Polyquaternium-7 | 0,225 | | | | 0,45 | | | | |
| Citric Acid | ad to pH 4,4-4,8 | ad to pH 4,4-4,8 | ad to pH 4,4-4,8 | ad to pH 4,4-4,8 | ad to pH 4,4-4,8 | ad to pH 4,4-4,8 | ad to pH 4,4-4,8 | | |
| Aqua | ad 100 | ad 100 | ad 100 | ad 100 | ad 100 | ad 100 | ad 100 | | |

| | | | | | | | | | |
|---|---|---|---|---|---|---|---|---|---|
| ¹Handelsprodukt: Compritol 888 CG ATO | | | | | | | | | |

## Patentansprüche

1. Kosmetische Reinigungszubereitung enthaltend
a) Lauryl Glucoside,
b) Sodium Methyl Cocoyl Taurate, und
c) Cocamidopropylbetaine
**dadurch gekennzeichnet, dass** die Reinigungszubereitung Glyceryl Behenate, Glyceryl Dibehenate und/oder Tribehenin enthält.

2. Reinigungszubereitung nach einem der vorhergehenden Ansprüche **dadurch gekennzeichnet, dass** der Anteil an Lauryl Glucoside von 1 Gew.-% bis 6 Gew.-%, bevorzugt von 1,5 Gew.-% bis 4,5 Gew.-% und insbesondere bevorzugt von 2,5 Gew.-% bis 3,5 Gew.-%, jeweils bezogen auf das Gesamtgewicht der Reinigungszubereitung, beträgt.

3. Reinigungszubereitung nach einem der vorhergehenden Ansprüche **dadurch gekennzeichnet, dass** der Anteil von Sodium Methyl Cocoyl Taurate von 0,25 Gew.-% bis 4 Gew.-%, bevorzugt von 0,5 Gew.-% bis 2,5 Gew.-% und insbesondere bevorzugt von 1 Gew.-% bis 2 Gew.-%, jeweils bezogen auf das Gesamtgewicht der Reinigungszubereitung, beträgt.

4. Reinigungszubereitung nach einem der vorhergehenden Ansprüche **dadurch gekennzeichnet, dass** Cocamidopropylbetaine in einem Anteil von 2 Gew.-% bis 8 Gew.-%, bevorzugt von 3 Gew.-% bis 7 Gew.-% und insbesondere bevorzugt von 4 Gew.-% bis 5 Gew.-%, jeweils bezogen auf das Gesamtgewicht der Reinigungszubereitung, enthalten ist.

5. Reinigungszubereitung nach einem der vorhergehenden Ansprüche **dadurch gekennzeichnet, dass** die Reinigungszubereitung einen pH-Wert im Bereich von 4,0 bis 5,5 und bevorzugt von 4,4 bis 4,8 aufweist.

6. Reinigungszubereitung nach einem der vorhergehenden Ansprüche **dadurch gekennzeichnet, dass** die Reinigungszubereitung keine mit Polyethylen Glykol substituierte Substanzen enthält.

7. Reinigungszubereitung nach einem der vorhergehenden Ansprüche **dadurch gekennzeichnet, dass** die Reinigungszubereitung keine Acrylat-basierten Polymere enthält.

8. Reinigungszubereitung nach einem der vorhergehenden Ansprüche **dadurch gekennzeichnet, dass** die Reinigungszubereitung keine zusätzlichen Polymere auf Basis von Polysacchariden und/oder Stärken enthält.

9. Reinigungszubereitung nach einem der vorhergehenden Ansprüche **dadurch gekennzeichnet, dass** Reinigungszubereitung kein Natriumlaurylethersulfat oder Natriumaurylsulfat enthält.

10. Reinigungszubereitung nach einem der vorhergehenden Ansprüche **dadurch gekennzeichnet, dass** Gesamtanteil aller enthaltenen Tenside von 3,25 Gew.-% bis 18 Gew.-%, weiterhin bevorzugt von 5 Gew.-% bis 14 Gew.-% und insbesondere bevorzugt von 7,5 Gew.-% bis 10,5 Gew.-%, jeweils bezogen auf das Gesamtgewicht der kosmetischen Reinigungszubereitung beträgt.

11. Reinigungszubereitung nach einem der vorhergehenden Ansprüche **dadurch gekennzeichnet, dass** diese Natriumsalicylat enthält und der Anteil von Natriumsalicylat bevorzugt von 0,1 Gew.-% bis 0,5 Gew.-% bezogen auf das Gesamtgewicht der Reinigungszubereitung beträgt.

12. Reinigungszubereitung nach einem der vorhergehenden Ansprüche **dadurch gekennzeichnet, dass** die Reinigungszubereitung Natirumbenzoat in einem Anteil von 0,1 Gew.-% bis 0,5 Gew.-% bezogen auf das Gesamtgewicht der Reinigungszubereitung enthält.

13. Reinigungszubereitung nach einem der vorhergehenden Ansprüche **dadurch gekennzeichnet, dass** die Reinigungszubereitung kein Natriumchlorid enthalten oder der Anteil von Natriumchlorid maximal 2,5 Gew.-% bezogen auf das Gesamtgewicht der jeweiligen kosmetischen Reinigungszubereitung beträgt.

14. Reinigungszubereitung nach einem der vorhergehenden Ansprüche **dadurch gekennzeichnet, dass** der Gesamtanteil von Glyceryl Behenate, Glyceryl Dibehenate und/oder Tribehenin von 1 Gew.-% bis 3 Gew.-%, bezogen auf das Gesamtgewicht der kosmetischen Reinigungszubereitung, beträgt.

## Claims

1. Cosmetic cleansing preparation comprising
a) Lauryl Glucoside,
b) Sodium Methyl Cocoyl Taurate, and
c) cocamidopropyl betaine,
**characterized in that** the cleansing preparation comprises Glyceryl Behenate, Glyceryl Dibehenate and/or Tribehenin.

2. Cleansing preparation according to any of the preceding claims, **characterized in that** the proportion of Lauryl Glucoside is from 1% by weight to 6% by weight, preferably from 1.5% by weight to 4.5% by weight and especially preferably from 2.5% by weight to 3.5% by weight, in each case based on the total weight of the cleansing preparation.

3. Cleansing preparation according to either of the preceding claims, **characterized in that** the proportion of Sodium Methyl Cocoyl Taurate is from 0.25% by weight to 4% by weight, preferably from 0.5% by weight to 2.5% by weight and especially preferably from 1% by weight to 2% by weight, in each case based on the total weight of the cleansing preparation.

4. Cleansing preparation according to any of the preceding claims, **characterized in that** cocamidopropyl betaine is present in a proportion from 2% by weight to 8% by weight, preferably from 3% by weight to 7% by weight and especially preferably from 4% by weight to 5% by weight, in each case based on the total weight of the cleansing preparation.

5. Cleansing preparation according to any of the preceding claims, **characterized in that** the cleansing preparation has a pH in the range from 4.0 to 5.5 and preferably from 4.4 to 4.8.

6. Cleansing preparation according to any of the preceding claims, **characterized in that** the cleansing preparation does not comprise any substances substituted with polyethylene glycol.

7. Cleansing preparation according to any of the preceding claims, **characterized in that** the cleansing preparation does not comprise any acrylate-based polymers.

8. Cleansing preparation according to any of the preceding claims, **characterized in that** the cleansing preparation does not comprise any additional polymers based on polysaccharides and/or starches.

9. Cleansing preparation according to any of the preceding claims, **characterized in that** the cleansing preparation does not comprise any sodium lauryl ether sulfate or sodium lauryl sulfate.

10. Cleansing preparation according to any of the preceding claims, **characterized in that** the total proportion of all surfactants present is from 3.25% by weight to 18% by weight, further preferably from 5% by weight to 14% by weight and especially preferably from 7.5% by weight to 10.5% by weight, in each case based on the total weight of the cosmetic cleansing preparation.

11. Cleansing preparation according to any of the preceding claims, **characterized in that** it comprises sodium salicylate and the proportion of sodium salicylate is preferably from 0.1% by weight to 0.5% by weight based on the total weight of the cleansing preparation.

12. Cleansing preparation according to any of the preceding claims, **characterized in that** the cleansing preparation comprises sodium benzoate in a proportion of 0.1% by weight to 0.5% by weight based on the total weight of the cleansing preparation.

13. Cleansing preparation according to any of the preceding claims, **characterized in that** the cleansing preparation does not comprise any sodium chloride or the proportion of sodium chloride is at most 2.5% by weight based on the total weight of the respective cosmetic cleansing preparation.

14. Cleansing preparation according to any of the preceding claims, **characterized in that** the total proportion of Glyceryl Behenate, Glyceryl Dibehenate and/or Tribehenin is from 1% by weight to 3% by weight, based on the total weight of the cosmetic cleansing preparation.

## Revendications

1. Préparation cosmétique de nettoyage, contenant
a) du laurylglucoside
b) du méthylcocoyltaurate de sodium et
c) des cocoamidopropylbétaïnes
**caractérisée en ce que** la préparation de nettoyage contient du béhénate de glycéryle, du dibéhénate de glycéryle et/ou de la tribéhénine.

2. Préparation de nettoyage selon l'une quelconque des revendications précédentes, **caractérisée en ce que** la proportion de laurylglucoside est de 1% en poids à 6% en poids, de préférence de 1,5% en poids à 4,5% en poids et en particulier de préférence de 2,5% en poids à 3,5% en poids, à chaque fois par rapport au poids total de la préparation de nettoyage.

3. Préparation de nettoyage selon l'une quelconque des revendications précédentes, **caractérisée en ce que** la proportion de méthylcocoyltaurate de sodium est de 0,25% en poids à 4% en poids, de préférence de 0,5% en poids à 2,5% en poids et en particulier de préférence de 1% en poids à 2% en poids, à chaque fois par rapport au poids total de la préparation de nettoyage.

4. Préparation de nettoyage selon l'une quelconque des revendications précédentes, **caractérisée en ce que** la cocoamidopropylbétaïne est contenue en une proportion de 2% en poids à 8% en poids, de préférence de 3% en poids à 7% en poids et en particulier de préférence de 4% en poids à 5% en poids, à chaque fois par rapport au poids total de la préparation de nettoyage.

5. Préparation de nettoyage selon l'une quelconque des revendications précédentes, **caractérisée en ce que** la préparation de nettoyage présente un pH dans la plage de 4,0 à 5,5 et de préférence de 4,4 à 4,8.

6. Préparation de nettoyage selon l'une quelconque des revendications précédentes, **caractérisée en ce que** la préparation de nettoyage ne contient pas de substances substituées par du polyéthylèneglycol.

7. Préparation de nettoyage selon l'une quelconque des revendications précédentes, **caractérisée en ce que** la préparation de nettoyage ne contient pas de polymères à base d'acrylate.

8. Préparation de nettoyage selon l'une quelconque des revendications précédentes, **caractérisée en ce que** la préparation de nettoyage ne contient pas de polymères supplémentaires à base de polysaccharides et/ou d'amidons.

9. Préparation de nettoyage selon l'une quelconque des revendications précédentes, **caractérisée en ce que** la préparation de nettoyage ne contient pas de lauryléthersulfate de sodium ou de laurylsulfate de sodium.

10. Préparation de nettoyage selon l'une quelconque des revendications précédentes, **caractérisée en ce que** la proportion totale de tous les tensioactifs contenus est de 3,25% en poids à 18% en poids, plus préférablement de 5% en poids à 14% en poids et en particulier de préférence de 7,5% en poids à 10,5% en poids, à chaque fois par rapport au poids total de la préparation de nettoyage.

11. Préparation de nettoyage selon l'une quelconque des revendications précédentes, **caractérisée en ce qu'**elle contient du salicylate de sodium et la proportion de salicylate de sodium est de préférence de 0,1% en poids à 0,5% en poids par rapport au poids total de la préparation de nettoyage.

12. Préparation de nettoyage selon l'une quelconque des revendications précédentes, **caractérisée en ce que** la préparation de nettoyage contient du benzoate de sodium en une proportion de 0,1% en poids à 0,5% en poids par rapport au poids total de la préparation de nettoyage.

13. Préparation de nettoyage selon l'une quelconque des revendications précédentes, **caractérisée en ce que** la préparation de nettoyage ne contient pas de chlorure de sodium ou contient une proportion de chlorure de sodium d'au maximum de 2,5% en poids par rapport au poids total de la préparation cosmétique de nettoyage respective.

14. Préparation de nettoyage selon l'une quelconque des revendications précédentes, **caractérisée en ce que** la proportion totale de béhénate de glycéryle, de dibéhénate de glycéryle et/ou de tribéhénine est de 1% en poids à 3% en poids, par rapport au poids total de la préparation cosmétique de nettoyage.
